(19) Europäisches Patentamt
European Patent Office
Office européen des brevets

(11) Publication number: 0 113 497
A1

(12) # EUROPEAN PATENT APPLICATION

(21) Application number: 83304979.4

(22) Date of filing: 30.08.83

(51) Int. Cl.³: H 01 B 3/24
H 01 F 27/12, C 07 C 21/12

(30) Priority: 31.08.82 US 413593

(43) Date of publication of application:
18.07.84 Bulletin 84/29

(84) Designated Contracting States:
BE DE FR GB IT SE

(71) Applicant: WESTINGHOUSE ELECTRIC CORPORATION
Westinghouse Building Gateway Center
Pittsburgh Pennsylvania 15222(US)

(72) Inventor: Palumbo, Anthony James
759 Richmond Drive
Hermitage Pennsylvania(US)

(72) Inventor: Kurz, Robert Anthony
4722 Sample Road
West Middlesex Pennsylvania(US)

(74) Representative: Marchant, James Ian et al,
Elkington and Fife High Holborn House 52/54 High Holborn
London WC1V 6SH(GB)

(54) **Perchloroethylene stabilized with aromatic phenols.**

(57) Method of inhibiting the attack by perchloroethylene on organic polymers subject to said attack, such as poly-para-xylylene, by adding to perchloroethylene from 0.01 to 0.5% by weight of an aromatic phenol stabilizer such as hydroquinone, di-tert-butyl paracresol, 8-hydroxy quinoline, resorcinol, phloroglucinol, pyrocatechol, pyrogallol, or mixtures thereof; particularly preferred is hydroquinone. Also an electrical apparatus containing an organic polymer subject to attack by perchloroethylene and the stabilizer.

EP 0 113 497 A1

Croydon Printing Company Ltd.

# METHOD OF PROTECTING ORGANIC POLYMERS FROM ATTACK BY PERCHLOROETHYLENE

This invention relates to a method of protecting organic polymers from attack by perchloroethylene.

When polychlorinated biphenyls were outlawed for use as dielectric fluids in electrical equipment on the grounds that they were an environmental hazard, a search was conducted for other suitable dielectric fluids. One of the substitute fluids that is now being used is perchloroethylene, $C_2Cl_4$. Perchloroethylene is a highly desirable dielectric fluid because it is not an environmental pollutant and yet has a high dielectric strength and is also non-flammable.

When percholoroethylene is manufactured, various stabilizers are put into it such as N-methyl pyrrole and p-tert amyl phenol (see U.S. Patent 4,293,433). These stabilizers are anti-oxidants which are added to prevent the decomposition of the perchloroethylene in the presence of oxygen.

While commercial perchlorethylene has good physical and electrical properties, it has been found that certain types of polymeric coatings used as insulation, such as parylene, are degraded by the perchloroethylene even with the commercial inhibitors present. The degradation of these organic polymers would eventually lead to electrical breakdown and failure of the transformer. The addition of common stabilizers such as melamine to the

commercially stabilized perchloroethylene dielectric fluid does not prevent the degradation of the polymeric coatings.

Accordingly, the present invention resides in a method of protecting organic polymers which are attacked by perchloroethylene from said attack which comprises adding to said perchloroethylene from 0.01 to 0.5% by weight of an aromatic phenol.

We have found that aromatic phenols substantially reduce the degradation of organic polymers, such as parylene, in the presence of perchloroethylene. This is indicated by a greatly reduced fall off in the dielectric strength when these stabilizers are present. In addition, while the commercial fluid oxidized to form suspended carbon particles, some of which settled on the transformer tank, this did not occur when the stabilizers of this invention were added to the fluid. As these particles are electrically conductive, they are highly undesirable in a dielectric fluid.

In order that the invention can be more clearly understood, a convenient embodiment thereof will now be described, by way of example, with reference to the accompanying drawing which is a side view, in section, of a transformer containing a stabilized dielectric fluid.

Referring to the drawing, a transformer 1 comprising a sealed tank 2, a ferrous metal coil 3 consisting of alternating layers of a conductor and an insulator, a primary coil 4, a secondary coil 5, and a dielectric fluid 6 which surrounds and covers the core and coils.

The stabilizers are aromatic phenols. Suitable aromatic phenols include hydroquinone, di-t-butylparacresol, 8-hydroxy-quinoline, resorcinol, phloroglucinol, pyrocatechol, and pyrogallol. The preferred stabilizer is hydroquinone as it has been found more effective in preventing the decomposition of the organic polymers in the presence of perchloroethlyene. At least 0.01% by weight of the stabilizer should be added to the commercially stabilized perchloroethylene as less than 0.01% has

been found to be marginal. More than 0.5% of the stabilizer can be used but that is undesirable as it has no additional effect in preventing the degradation of the polymers. The preferred percentage is from 0.05 to 0.1%.

The stabilizers may be used in combination with perchloroethylene alone or in combination with a mixture of perchloroethylene and another dielectric fluid. For example, perchloroethylene is often used in combination with up to 30%, preferably from 5 to 30%, mineral oil. The fluid may be used in all sorts of electrical apparatus including transformers, capacitors, cables, and circuit breakers which contain organic polymers attackable by perchloroethylene. Parylene is poly (para-xylene), and comes in two forms, parylene "C"

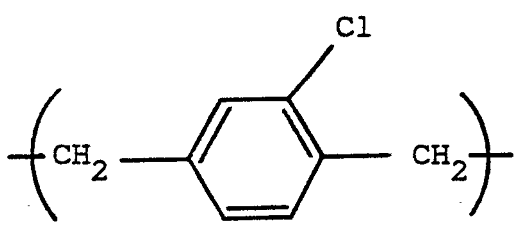 $n > 5000$

and parylene "N"

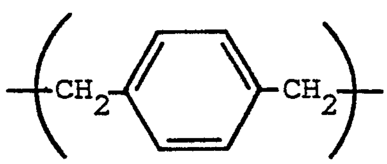 $n > 5000$.

Parylene "C" is preferred as its properties are superior. Other organic polymers such as polyesters, epoxies, and polyester-amide-imides may also be attacked by perchloroethylene, depending on how they are prepared.

The invention will now be illustrated with reference to the following Examples:

EXAMPLE

Tests were performed to determine the stability of perchloroethylene alone and when mixed with mineral oil after being heated for seven days at 125°C. The perchloroethylene used in these tests was purchased from the Diamond Shamrock Company and was analyzed as containing 55 ± 5 ppm of N-methyl pyrrole as an antioxidant. The dielectric fluids were sealed in glass tubes and the dissipation factor was measured at the end of the test. The following table gives the results.

| Dielectric Fluid | Dissipation Factor, % |
|---|---|
| Neat $C_2Cl_4$ | 0.5 |
| Neat $C_2Cl_4$ + core steel | 0.6 |
| Neat mineral oil | 0.3 |
| 75% $C_2Cl_4$/25% mineral oil | 4.8 |

The above table shows that a mixture of perchloroethylene and mineral oil was not stable under these conditions.

Tests were performed to determine whether or not the increase in the dissipation factor which resulted above when mineral oil was mixed with perchloroethylene was due to the presence of oxygen. Samples of dielectric fluids were prepared and heated at 125°C under various conditions for seven days and then tested for dissipation factor. The following table gives the results.

| Dielectric Fluid | Dissipation Factor, % |
| --- | --- |
| $C_2Cl_4$, with air | 0.01 |
| $C_2Cl_4$, with no air | 0.40 |
| $C_2Cl_4$ nitrogen covered, no air | 0.006 |
| 75% $C_2Cl_4$/25% mineral oil, with air | 10.7 |
| 75% $C_2Cl_4$/25% mineral oil, nitrogen covered, no air | 10.4 |
| 75% $C_2Cl_4$/25% mineral oil, as is -- no heating | 0.03 |

The above table shows that the increase in dissipation factor of the perchloroetheylene-mineral oil mixture was not due to the presence of oxygen.

Tests were then performed on a 75% $C_2Cl_4$/25% mineral oil mixture containing various stabilizers. The fluids were heated for seven days at 125°C and then tested for dissipation factor. The following table gives the results.

| Stabilizer | Dissipation Factor, % |
|---|---|
| None | 4.3 |
| 0.05% di-t-butyl-p-cresol (DBPC) | 3.2 |
| 2% of a 17% - 1% mixture of DBPC + 8-hdroxy-quinoline | 3.0 |
| .15% DBPC + 2% of a 17% - 1% mixture of DBPC + 8-hydroxy-quninoline | 3.7 |
| 0.1% hydroquinone | 0.3 |
| 0.5% hydroquinone | 0.3 |
| 0.1% hydroquinone + 0.15% DBPC + 2% of a 17% - 1% mixture of DBPC + 8-hydroxy-quinoline | 1.2 |

The above table shows the effectiveness of hydroquinone in preventing an increase in dissipation factor.

EXAMPLE 2

Tests were performed on a 75% $C_2Cl_4$/25% mineral oil mixture containing various concentrations of hydroquinone. The dissipation factor of the fluids was measured before and after aging for seven days in 125°C. The following table gives the results.

| | Dissipation Factor | |
|---|---|---|
| Hydroquinone Concentration | Before Aging | After Aging |
| 0.01% | 0.03 | 0.93 |
| 0.05 | 0.03 | 1.9 |
| 0.10 | 0.04 | 0.3 |
| 0.50 | 0.03 | 0.3 |
| 0 | 0.05 | 3.7 |

The above table shows that hydroquinone is effective in preventing an increase in dissipation factor at a concentration range of about 0.01% to about 0.5%.

EXAMPLE 3

0.05% of various stablizers were added to a mixture of 75% $C_2Cl_4$/25% mineral oil. The dissipation factor of the fluids was measured before and after aging for seven days in 125°C. The following table gives the results.

| | Dissipation Factor, % | |
|---|---|---|
| Stabilizer | Before Aging | After Aging |
| None | 0.03-0.07 | 4.0-10.0 |
| Hydroquinone | 0.03-0.07 | 0.3-1.9 |
| Resorcinol | 0.1 | 0.3 |
| Phloroglucinol | 0.06 | 0.85 |
| Pyrocatechol | 0.4 | 3.2 |
| Pyrogallol | 0.1 | 2.1 |
| Anthraquinone | 0.06 | 5.6 |

The above table shows the effectiveness of various aromatic phenols in preventing an increase in dissipation factor.

EXAMPLE 4

Aluminum strips two inches wide by 5 mils thick were coated with .8 mils of parylene "C" sold by Union Carbide. The coated strips had a dielectric strength of 4470 volts per mil. The strips were placed in filled containers containing Diamond Shamrock perchloroethylene for various lengths of time at 125°C, and then were again tested for dielectric strength. The following table gives the results.

| STABILIZER | TIME | TEST RESULTS |
|---|---|---|
| None | 7 days | polymer decomposed no dielectric reading possible |
| 0.05% Hydroquinone | 42 days | 84.5% of dielectric strength retained |

Claims:

1. A method of protecting organic polymers which are attacked by perchloroethylene from said attack characterized by adding to said perchloroethylene from 0.01 to 0.5% by weight of an aromatic phenol.

2. A method according to claim 1, characterized by the organic polymer is parylene.

3. A method according to claim 1 or 2, characterized by the perchloroethylene is mixed with mineral oil, said mineral oil being from 5 to 30% of said mixture.

4. A method according to claim 1, 2 or 3, characterized by the aromatic phenol is hydroquinone, di-tert-butyl paracresol, 8-hydroxy quinoline, resorcinol, phloroglucinol, pyrocatechol, pyrogallol, or mixtures thereof.

5. A method according to any of claims 1 to 4, characterized by the amount of the aromatic phenol is from 0.05 to 0.1%.

6. Electrical apparatus containing perchloroethylene dielectric fluid in contact with an organic polymer characterized in that said polymer is protected against attack by said perchloroethylene, by a method as claimed in any of the preceding claims.

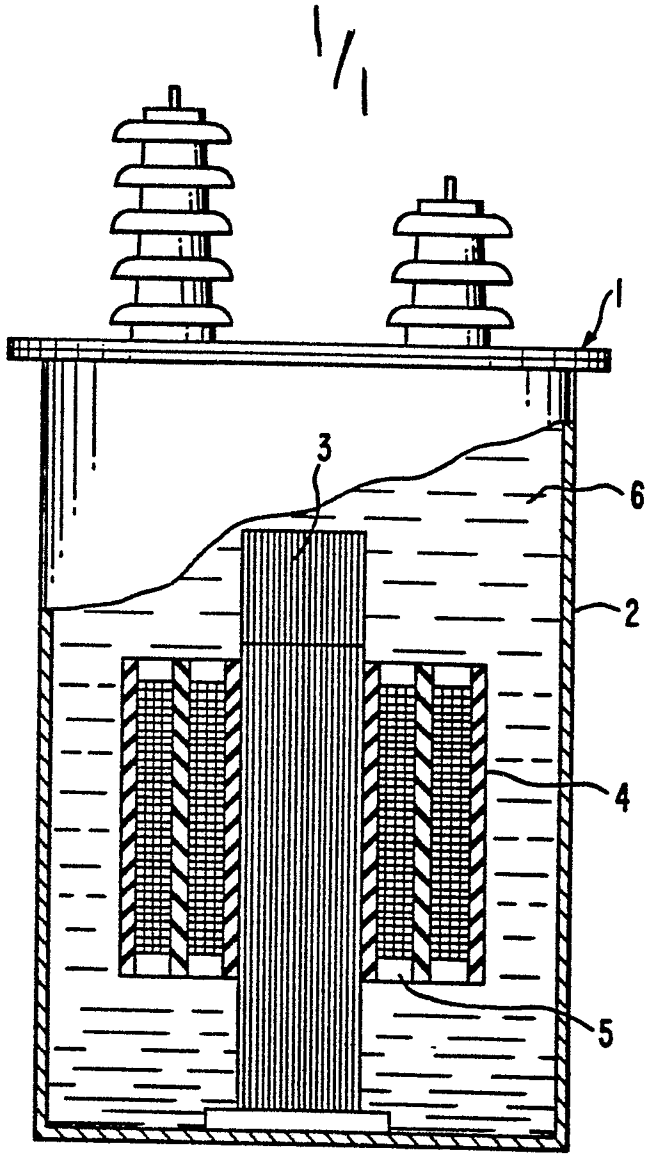
1
3
6
2
4
5

European Patent Office

# EUROPEAN SEARCH REPORT

0113497

Application number

EP 83304979.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X,D<br>Y | US - A - 4 293 433 (BORROR et al.)<br>* Column 2, lines 40-68; table II; column 5, lines 22-25 *<br>-- | 1,4-6,<br>3 | H 01 B 3/24<br>H 01 F 27/12<br>C 07 C 21/12 |
| Y | EP - A1 - 0 037 280 (WESTINGHOUSE)<br>* Page 4, lines 14-20; example 8; abstract *<br>-- | 3 | |
| X,E<br>P | EP - A1 - 0 083 736 (SIEMENS)<br>* Claims 6,7 *<br>-- | 1,4,6 | |
| A | AT - B - 144 024 (ROESSLER)<br>* Claims *<br>-- | 1,4 | |
| A | K. STOECKHERT "Kunststoff-Lexikon" Carl-Hanser-Verlag, München, Wien, 7th edition, 1981<br>pages 408,409<br>* Catchword "Poly-p-Xylylen" *<br>---- | 2 | TECHNICAL FIELDS SEARCHED (Int. Cl. 3)<br>H 01 B 3/00<br>H 01 F 27/00<br>C 07 C |

The present search report has been drawn up for all claims

| Place of search | Date of completion of the search | Examiner |
|---|---|---|
| VIENNA | 25-11-1983 | KUTZELNIGG |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document